(19) Europäisches Patentamt
European Patent Office
Office européen des brevets

(11) **EP 3 484 847 B1**

(12) **EUROPÄISCHE PATENTSCHRIFT**

(45) Veröffentlichungstag und Bekanntmachung des Hinweises auf die Patenterteilung:
**28.10.2020   Patentblatt 2020/44**

(21) Anmeldenummer: **17742222.7**

(22) Anmeldetag: **17.07.2017**

(51) Int Cl.:
*C07C 51/44* *(2006.01)*       *C07C 51/48* *(2006.01)*
*C07C 53/02* *(2006.01)*

(86) Internationale Anmeldenummer:
**PCT/EP2017/068055**

(87) Internationale Veröffentlichungsnummer:
**WO 2018/015352 (25.01.2018 Gazette 2018/04)**

(54) **VERFAHREN ZUM ABSONDERN VON AMEISENSÄURE AUS EINEM REAKTIONSGEMISCH DURCH EXTRAKTION**

METHOD FOR SEPARATING FORMIC ACID FROM A REACTION MIXTURE BY MEANS OF EXTRACTION

PROCÉDÉ PERMETTANT DE SÉPARER PAR EXTRACTION DE L'ACIDE FORMIQUE PRÉSENT DANS UN MÉLANGE RÉACTIONNEL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2016   DE 102016213100**

(43) Veröffentlichungstag der Anmeldung:
**22.05.2019   Patentblatt 2019/21**

(73) Patentinhaber: **OxFA GmbH**
**96110 Scheßlitz (DE)**

(72) Erfinder:
• **JBACH, Hermann Wolf**
**96120 Bischberg (DE)**
• **KOHLER, Florian**
**90419 Nürnberg (DE)**
• **SCHMIDT, Matthias**
**91362 Pretzfeld (DE)**
• **SCHOLZ, Gunthard**
**96163 Gundelsheim (DE)**
• **DIRAUF, Martin**
**96250 Ebensfeld (DE)**

(74) Vertreter: **Dr. Gassner & Partner mbB**
**Wetterkreuz 3**
**91058 Erlangen (DE)**

(56) Entgegenhaltungen:
**WO-A1-2013/092403      DE-A1-102011 077 232**

EP 3 484 847 B1

**Beschreibung**

Die Erfindung betrifft ein Verfahren zum Absondern von Ameisensäure aus einem Reaktionsgemisch durch Extraktion, wobei das Reaktionsgemisch neben der Ameisensäure ein Polyoxometallat-Ion der allgemeinen Formel $[PMo_xV_yO_{40}]^{n-}$ als Katalysator und ein den Katalysator lösendes Lösungsmittel umfasst, wobei $6 \leq x \leq 11$, $1 \leq y \leq 6$, $x + y = 12$ und $3 < n < 10$, wobei n, x und y jeweils eine ganze Zahl ist. Dabei erfolgt das Absondern durch eine Extraktion mittels eines die Ameisensäure und den Katalysator extrahierenden polaren organischen Extraktionsmittels, welches beim Mischen mit dem Lösungsmittel eine Phasengrenze zwischen dem Lösungsmittel und dem Extraktionsmittel bildet.

Aus der DE 10 2011 077 232 A1 und der dazu korrespondierenden EP 2 473 467 B1 ist ein Verfahren zur katalytischen Erzeugung von Ameisensäure bekannt, wobei ein als Katalysator dienendes Polyoxometallat-Ion der allgemeinen Formel $[PMo_xV_yO_{40}]^{5-}$ bei einer Temperatur unterhalb von 120 °C mit einem alpha-Hydroxyaldehyd, einer alpha-Hydroxycarbonsäure, einem Kohlenhydrat oder einem Glycosid in einer flüssigen Lösung in Kontakt gebracht wird, wobei $6 \leq x \leq 11$ und $1 \leq y \leq 6$ und $x + y = 12$, wobei x und y jeweils eine ganze Zahl ist. Weiterhin ist in der Druckschrift beschrieben, dass die so hergestellte Ameisensäure mittels Extraktion abgesondert werden kann. Zur Extraktion der Ameisensäure kann als Extraktionsmittel ein Ether eingesetzt werden. Neben der Ameisensäure kann der Katalysator zusammen mit der Ameisensäure mittels eines Amids extrahiert werden.

Aufgabe der vorliegenden Erfindung ist es, ein alternatives Verfahren zum Absondern von Ameisensäure aus einem Reaktionsgemisch durch Extraktion anzugeben.

Die Aufgabe wird durch die Merkmale des Patentanspruchs 1 gelöst. Zweckmäßige Ausgestaltungen ergeben sich aus den Merkmalen der Patentansprüche 2 bis 13.

Erfindungsgemäß ist ein Verfahren zum Absondern von Ameisensäure aus einem Reaktionsgemisch durch Extraktion vorgesehen, wobei das Reaktionsgemisch neben der Ameisensäure ein Polyoxometallat-Ion der allgemeinen Formel $[PMo_xV_yO_{40}]^{n-}$ als Katalysator und ein den Katalysator lösendes Lösungsmittel umfasst, wobei $6 \leq x \leq 11$, $1 \leq y \leq 6$, $x + y = 12$ und $3 < n < 10$, wobei n, x und y jeweils eine ganze Zahl ist. Dabei erfolgt das Absondern durch eine Extraktion, insbesondere eine Reaktivextraktion, mittels eines die Ameisensäure und den Katalysator extrahierenden polaren organischen Extraktionsmittels, welches beim Mischen mit dem Lösungsmittel eine Phasengrenze zwischen dem Lösungsmittel und dem Extraktionsmittel bildet. Bei der Reaktivextraktion reagiert das Extraktionsmittel chemisch zumindest mit der Ameisensäure oder dem Katalysator, beispielsweise in einer Ionenaustauschreaktion oder durch Ausbilden chemischer Bindungen.

Das Lösungsmittel kann ein Lösungsmittel sein, welches neben dem Katalysator auch ein Substrat des Katalysators löst. Bei dem Substrat des Katalysators kann es sich um ein alpha-Hydroxyaldehyd, eine alpha-Hydroxycarbonsäure, ein Kohlenhydrat, ein Glycosid oder ein eine Kohlenstoffkette enthaltendes Polymer mit mindestens einer wiederholt als Substituent an die Kohlenstoffkette gebundenen OH-Gruppe und/oder mit einem wiederholt in der Kohlenstoffkette enthaltenen O-, N- oder S-Atom handeln. Unter einem alpha-Hydroxyaldehyd wird jedes Molekül verstanden, bei welchem eine OH-Gruppe direkt an einem C-Atom gebunden ist, wobei an dem C-Atom auch direkt das C-Atom einer Aldehyd-Gruppe gebunden ist. Unter einer alpha-Hydroxycarbonsäure wird jedes Molekül verstanden, bei welchem eine OH-Gruppe direkt an einem C-Atom gebunden ist, wobei an dem C-Atom auch direkt das C-Atom einer Carboxy-Gruppe gebunden ist. Unter einem alpha-Hydroxyaldehyd und unter einer alpha-Hydroxycarbonsäure kann auch jede Substanz verstanden werden, welche ein alpha-Hydroxyaldehyd oder eine alpha-Hydroxycarbonsäure enthält. Wenn das Substrat selbst in flüssiger Form vorliegt, kann auch das Substrat das den Katalysator lösende Lösungsmittel sein.

Bei dem Extraktionsmittel handelt es sich um N-(n-Hexadecyl)formamid, N-Di-n-acetamid oder ein N,N-Dialkylcarbonsäureamid, welches für eine Extraktion des in einer Konzentration von 1,5 Gew.-% in Wasser enthaltenen Katalysators bei 40 °C einen Verteilungskoeffizienten für den Katalysator aufweist, welcher mindestens um den Faktor 7, insbesondere mindestens um den Faktor 8, insbesondere mindestens um den Faktor 9, insbesondere mindestens um den Faktor 10, insbesondere mindestens um den Faktor 15, insbesondere mindestens um den Faktor 20, insbesondere mindestens um den Faktor 25, insbesondere mindestens um den Faktor 30, größer ist als ein Verteilungskoeffizient für eine Extraktion der in einer Konzentration von 5 Gew.-% in Wasser enthaltenen Ameisensäure bei 40 °C. Der Verteilungskoeffizient K ist wie folgt definiert:

$$K = \frac{(Konzentration\ Ameisens\ddot{a}ure\ bzw.\ Katalysator)\ im\ Extraktionsmittel}{(Konzentration\ Ameisens\ddot{a}ure\ bzw.\ Katalysator)\ im\ Wasser}$$

Mit "Wasser" kann hier auch die wässrige Phase in einem zweiphasigen System mit dem Extraktionsmittel gemeint sein. Die wässrige Phase kann bei der Bestimmung des Verteilungskoeffizienten für den Katalysator beispielsweise auch Ameisensäure enthalten. Das Extraktionsmittel kann dadurch den Katalysator am Beginn der Extraktion schneller extrahieren als die Ameisensäure. "Schneller" bedeutet dabei insbesondere, dass pro Zeiteinheit ein größerer Gewichtsprozentanteil vom Katalysator an dem insgesamt im Reaktionsgemisch enthaltenen Katalysator als von der Ameisensäure an der insgesamt im Reaktionsgemisch enthaltenen Ameisensäure in das Extraktionsmittel übergeht.

Gemäß Schierbaum B. et al., "Integriertes Verfahren zur Reinigung von carbonsäurehaltigen Prozeßabwässern", Chemie Ingenieur Technik (69), 1997, Seiten 519 bis 523, beträgt der Verteilungskoeffizient $K$ von Ameisensäure in einem zweiphasigen System aus N,N-Di-n-butylformamid (DBFA) und 5 % Ameisensäure in Wasser bei einer Temperatur von 40 °C 2,5. Für den Katalysator $[PMo_7V_5O_{40}]^{8-}$ (HPA-5) zeigen Messungen der Erfinder in einem zweiphasigen System mit DBFA und 1,5 % HPA-5 in Wasser einen Verteilungskoeffizienten $K$ von 3284. Dies zeigt, dass in diesem Beispiel der Verteilungskoeffizient für die Extraktion des Katalysators um den Faktor 1313 größer ist als der Verteilungskoeffizient für die Extraktion der Ameisensäure.

Das Extraktionsmittel wird vor der Extraktion mit dem Katalysator gesättigt. Dadurch wird die Extraktion des Katalysators bei der Extraktion unterbunden bzw. erreicht, dass bei der Extraktion nur die Ameisensäure extrahiert wird.

Alternativ wird der zusammen mit der Ameisensäure extrahierte Katalysator aus dem eingesetzten Extraktionsmittel nach der Extraktion mittels Fällung als Salz oder mittels einer weiteren Extraktion mit einem polaren weiteren Extraktionsmittel und einem Temperaturwechsel des Extraktionsmittels und/oder einer Erhöhung des pH-Werts des Extraktionsmittels abgesondert und wieder dem Reaktionsgemisch zugeführt. Dadurch kann eine wesentliche Verminderung der Konzentration des Katalysators in dem Reaktionsgemisch vermieden und eine durch den Katalysator katalysierte Reaktion in dem Reaktionsgemisch aufrechterhalten werden. Dazu kann z. B. zunächst die Ameisensäure durch Destillation und danach der Katalysator durch Fällung als Salz vom Extraktionsmittel abgesondert werden.

Durch das erfindungsgemäße Verfahren ist es möglich, die Ameisensäure zu extrahieren und gleichzeitig die Konzentration des Katalysators in dem Reaktionsgemisch zumindest im Wesentlichen aufrecht zu erhalten. Dadurch kann die Reaktion zur Erzeugung der Ameisensäure in dem Reaktionsgemisch weiter ablaufen. Sie wird durch die Extraktion der Ameisensäure zumindest nicht wesentlich behindert.

Bei dem bisher bekannten Extraktionsverfahren, bei dem der Katalysator zusammen mit der Ameisensäure extrahiert wurde, wurde der Katalysator dem Reaktionsgemisch entzogen und stand dann nicht mehr für eine weitere Reaktion zur Verfügung. Da der Katalysator verhältnismäßig teuer ist, wird das Verfahren durch den Verlust des Katalysators und der damit einhergehenden Notwendigkeit, dem Reaktionsgemisch neuen Katalysator zuzuführen, verhältnismäßig teuer, obwohl der Katalysator nur in geringer Menge benötigt wird.

Die Extraktion kann zweistufig durchgeführt werden, indem das Reaktionsgemisch in einem ersten Schritt der Extraktion mit einer ersten Menge des Extraktionsmittels, insbesondere für eine erste Zeit, zur Extraktion des Katalysators und in einem zweiten Schritt der Extraktion mit einer zweiten Menge des Extraktionsmittels, insbesondere für eine zweite Zeit, zur Extraktion der Ameisensäure extrahiert wird. Bei der ersten Menge kann es sich um eine erste und bei der zweiten Menge um eine zweite Teilmenge des insgesamt eingesetzten Extraktionsmittels handeln. Die Menge des insgesamt bei dem Verfahren eingesetzten Extraktionsmittels kann von der 0,1-fachen bis zur dreifachen Menge des Reaktionsgemischs, jeweils bezogen auf das Gewicht, betragen. Der in dem ersten Schritt der Extraktion extrahierte Katalysator wird wieder dem Reaktionsgemisch zugeführt. Der Katalysator aus dem in dem ersten Schritt der Extraktion eingesetzten Extraktionsmittel kann nach dem ersten Schritt der Extraktion mittels Fällung als Salz oder mittels einer weiteren Extraktion mit einem polaren weiteren Extraktionsmittel und einem Temperaturwechsel des in dem ersten Schritt der Extraktion eingesetzten Extraktionsmittels und/oder einer Erhöhung des pH-Werts des in dem ersten Schritt der Extraktion eingesetzten Extraktionsmittels abgesondert werden.

Durch den Temperaturwechsel und/oder die Erhöhung des pH-Werts kann der in dem Extraktionsmittel enthaltene Katalysator in das weitere Extraktionsmittel ausgetrieben werden.

Das weitere Extraktionsmittel bildet beim Mischen mit dem Extraktionsmittel oder dem in dem ersten Schritt der Extraktion eingesetzten Extraktionsmittel eine Phasengrenze zwischen dem weiteren Extraktionsmittel und dem Extraktionsmittel oder dem in dem ersten Schritt der Extraktion eingesetzten Extraktionsmittel.

Aus dem in dem zweiten Schritt der Extraktion eingesetzten Extraktionsmittel kann die Ameisensäure z. B. mittels Destillation oder durch Fällung als Formiat abgesondert werden.

Gegenüber der sowohl den Katalysator als auch die Ameisensäure extrahierenden einstufigen Extraktion hat dieses Verfahren den Vorteil, dass es eine vollständige oder zumindest nahezu vollständige Extraktion des Katalysators aus dem Reaktionsgemisch in dem ersten Schritt erlaubt und in dem zweiten Schritt nur noch oder zumindest nahezu nur noch Ameisensäure in das Extraktionsmittel übertritt. Das Verfahren kann es ersparen, mit dem Extrakt einen weiteren Trennschritt durchzuführen, um den Katalysator von der Ameisensäure zu trennen.

Die erste Zeit und die zweite Zeit sowie die erste Menge des Extraktionsmittels und die zweite Menge des Extraktionsmittels können jeweils identisch sein. Eine möglichst geringe Absonderung von Ameisensäure aus dem Reaktionsgemisch in dem ersten Schritt der Extraktion und damit gegebenenfalls ein möglichst geringer Verlust von Ameisensäure kann jedoch erreicht werden, wenn die zweite Zeit länger ist als die erste Zeit und/oder die erste Menge kleiner ist als die zweite Menge, insbesondere wenn die erste Zeit besonders kurz und/oder die erste Menge besonders klein sind/ist. In jedem Fall sollte die erste Zeit und die erste Menge jedoch ausreichend bemessen sein, um mehr als 33 %, insbesondere mehr als 50 %, insbesondere mehr als 80 % des jeweils zu extrahierenden Katalysators aus dem Reaktionsgemisch extrahieren zu können oder um sogar eine vollständige oder nahezu vollständige Extraktion des Katalysators aus dem Reaktionsgemisch zu ermöglichen. Die Absonderung relativ reiner Ameisensäure aus dem in dem zweiten Schritt der Extraktion eingesetzten Extraktionsmittel ist bei einer im ersten Schritt erfolgten vollständigen oder nahezu vollständigen Extraktion des Katalysators aus dem Reaktionsgemisch verhältnismäßig einfach, weil die Ameisensäure dann in dem im zweiten Schritt eingesetzten Extraktionsmittel ohne den oder zumindest nahezu ohne den Katalysator vorliegt.

Der Katalysator kann mittels der Fällung als Salz gleichzeitig mit einer Fällung der zusätzlich extrahierten Ameisensäure als Formiat abgesondert werden.

Es ist auch möglich, dass das polare weitere Extraktionsmittel das Lösungsmittel, beispielsweise Wasser, ist und/oder die Erhöhung des pH-Werts des Extraktionsmittels oder des in dem ersten Schritt der Extraktion eingesetzten Extraktionsmittels durch Zusatz eines Carbonats und/oder eines Hydroxids erfolgt.

Die Fällung als Salz kann mittels eines Hydroxids, insbesondere KOH oder NaOH, oder einer sonstigen Base erfolgen.

Das gefällte Salz kann dem Reaktionsgemisch zugeführt werden und davor in dem Lösungsmittel gelöst werden und, insbesondere mittels Ameisensäure, auf einen pH-Wert des Reaktionsgemischs eingestellt werden. Alternativ oder zusätzlich kann das weitere Extraktionsmittel nach der weiteren Extraktion dem Reaktionsgemisch zugeführt werden und davor, insbesondere mittels Ameisensäure, auf einen pH-Wert des Reaktionsgemischs eingestellt werden.

Wenn der Katalysator aus dem Extraktionsmittel nach der Extraktion oder aus dem in dem ersten Schritt der Extraktion eingesetzten Extraktionsmittel nach dem ersten Schritt der Extraktion mittels der weiteren Extraktion mit dem polaren weiteren Extraktionsmittel, insbesondere dem Lösungsmittel, und einem Temperaturwechsel und/oder einer Erhöhung des pH-Werts, insbesondere durch Zugabe eines Carbonats und/oder eines Hydroxids, abgesondert wird, um ihn wieder dem Reaktionsgemisch zuzuführen, kann dem Extraktionsmittel vor der Extraktion oder vor der weiteren Extraktion oder zumindest dem in dem ersten Schritt der Extraktion eingesetzten Extraktionsmittel vor dem ersten Schritt der Extraktion oder vor der weiteren Extraktion ein, insbesondere unpolares, Additiv zugesetzt werden. Dies erleichtert die weitere Extraktion durch Temperaturwechsel und/oder Erhöhung des pH-Werts. Das Additiv bewirkt eine andere Verteilung zwischen einer organischen und einer anorganischen Phase bei Temperaturänderung oder Änderung des pH-Werts. Bei dem Additiv kann es sich um Petroleum, eine Fraktion von Petroleum, n-Hexan, n-Oktan, n-Decan, Oleylalkohol, Toluol, Dibutylether oder Tri-n-butylphosphat handeln.

Bei dem Lösungsmittel kann es sich um ein protisches und/oder polares Lösungsmittel, insbesondere Wasser oder ein mittels des Katalysators umsetzbares Substrat, handeln.

Eine Möglichkeit, die Extraktion von Ameisensäure in dem ersten Schritt der Extraktion zu unterbinden bzw. zu erreichen, dass bei der Extraktion oder in dem ersten Schritt der Extraktion nur der Katalysator extrahiert wird, besteht darin, das eingesetzte Extraktionsmittel vor der Extraktion mit Ameisensäure zu sättigen. Dabei wurde überraschenderweise festgestellt, dass die Reaktivextraktion des Katalysators mit einem mit Ameisensäure gesättigten N,N-Dialkylcarbonsäureamid als Extraktionsmittel effektiver ist als mit dem nicht mit Ameisensäure gesättigten N,N-Dialkylcarbonsäureamid.

Bei dem N,N-Dialkylcarbonsäureamid als Extraktionsmittel kann es sich um Dipentylformamid, N,N-Di-n-butylformamid, N-methyl-N-heptylformamid, N-n-butyl-N-2-ethylhexylformamid oder N-n-butyl-N-cyclohexylformamid handeln. Insbesondere N,N-Di-n-butylformamid hat sich als sehr gut geeignet erwiesen. N,N-Dialkylcarbonsäureamide eignen sich besonders gut zur Reaktivextraktion des Katalysators.

Um eine in dem Reaktionsgemisch erfolgende Reaktion zur Bildung von Ameisensäure durch die Extraktion nicht unterbrechen zu müssen, kann die Extraktion jeweils nur mit einer Teilmenge des Reaktionsgemischs durchgeführt werden, die nach der Extraktion wieder dem restlichen Reaktionsgemisch zugeführt wird. Dadurch kann auch verhindert werden, dass der Gehalt an Ameisensäure in dem Reaktionsgemisch soweit ansteigt, dass er die Bildung von weiterer Ameisensäure hemmen würde. Eine Hemmung der Bildung der Ameisensäure würde jedoch auch eintreten, wenn der pH-Wert des Reaktionsgemischs zu weit ansteigen würde. Bei der Extraktion oder dem zweiten Schritt der Extraktion sollte daher nur so viel Ameisensäure aus dem Reaktionsgemisch extrahiert werden, dass der pH-Wert des Reaktionsgemischs nicht über 3, insbesondere nicht über 2,5, insbesondere nicht über 2, steigt. Der extrahierte Katalysator kann dem Reaktionsgemisch zugeführt werden, indem er zunächst der Teilmenge des Reaktionsgemischs zugeführt wird, die dann ihrerseits dem restlichen Reaktionsgemisch zugeführt wird.

Um die Ameisensäure von dem in der Extraktion oder dem zweiten Schritt der Extraktion eingesetzten Extraktionsmittel abzusondern, kann eine Destillation, beispielsweise eine Flash-Destillation, oder eine Fällung als Formiat oder ein beliebiges anderes Verfahren zur Absonderung von Ameisensäure aus dem Extraktionsmittel durchgeführt werden. Nach der Absonderung des Katalysators und der Ameisensäure aus dem Extraktionsmittel kann das Extraktionsmittel wiederverwendet werden. Insbesondere ist es möglich, das Extraktionsmittel in einem kontinuierlichen Prozess im Kreis zu führen.

Das erfindungsgemäße Verfahren kann auch eine katalytische Erzeugung von Ameisensäure mittels des Katalysators und eine Regeneration des dabei reduzierten Katalysators umfassen, wobei der Katalysator bei einer Temperatur oberhalb von 70 °C, 80 °C oder 90 °C und unterhalb von 160 °C, 150 °C oder 140 °C, insbesondere unterhalb 120 °C, mit einem alpha-Hydroxyaldehyd, einer alpha-Hydroxycarbonsäure, einem Kohlenhydrat, einem Glycosid oder einem eine Kohlenstoffkette enthaltenden Polymer mit mindestens einer wiederholt als Substituent an die Kohlenstoffkette gebundenen OH-Gruppe und/oder mit einem wiederholt in der Kohlenstoffkette enthaltenen O-, N- oder S-Atom als Substrat in dem Reaktionsgemisch in Kontakt gebracht wird. Der dabei reduzierte Katalysator kann durch Oxidation wieder in seinen Ausgangszustand versetzt werden, wobei das Reaktionsgemisch dazu mit einem einen Volumenanteil von mindestens 18 %, insbesondere mindestens 19 %, insbesondere mindestens 20 %, Sauerstoff enthaltenden Gas bei einem Gasdruck von mindestens 2 bar, insbesondere mindestens 3 bar, insbesondere mindestens 4 bar, insbesondere mindestens 5 bar, insbesondere mindestens 6 bar, insbesondere mindestens 7 bar, insbesondere mindestens 8 bar, insbesondere mindestens 9 bar, insbesondere mindestens 10 bar, insbesondere mindestens 11 bar, insbesondere mindestens 12 bar, insbesondere mindestens 13 bar, und höchstens 33 bar, insbesondere höchstens 28 bar, insbesondere höchstens 24 bar, insbesondere höchstens 19 bar, insbesondere höchstens 18 bar, insbesondere höchstens 17 bar, insbesondere höchstens 16 bar, insbesondere höchstens 15 bar, insbesondere höchstens 14 bar, insbesondere höchstens 13 bar, mittels einer Mischvorrichtung oder über eine flüssigkeitsundurchlässige gasdurchlässige Membran in Kontakt gebracht wird. Die Mischvorrichtung kann einen statischen Mischer, eine Reaktionsmischpumpe, eine Düse, insbesondere eine Venturi-Düse oder eine Sprühdüse, und/oder einen Gaseintragsrührer umfassen. Die Mischvorrichtung kann dazu aus mindestens einer der genannten Mischeinrichtungen bestehen oder eine Mehrzahl davon oder auch eine Mehrzahl verschiedener der genannten Mischeinrichtungen umfassen.

Die Erfinder der vorliegenden Patentanmeldung haben festgestellt, dass das bei der katalytischen Umsetzung der genannten Substrate bei einem limitierten Druck entstehende CO und/oder $CO_2$ einen unerwartet stark limitierenden Einfluss auf die Ausbeute an Ameisensäure und/oder die Geschwindigkeit der Erzeugung von Ameisensäure hat. Sie haben weiterhin festgestellt, dass die Geschwindigkeit der Erzeugung von Ameisensäure und/oder die Ausbeute an Ameisensäure dabei unerwartet deutlich gesteigert werden kann, wenn bei der Reaktion entstehendes und in das Gas übergehendes CO und/oder $CO_2$ in einer solchen Menge abgeführt wird, dass ein Volumenanteil von CO und $CO_2$ zusammen an dem Gas 80 %, insbesondere 70 %, insbesondere 60 %, insbesondere 55 %, insbesondere 50 %, insbesondere 45 %, insbesondere 40 %, insbesondere 35 %, insbesondere 30 %, insbesondere 25 %, insbesondere 20 %, nicht übersteigt. Das Verfahren kann dadurch mit einem verhältnismäßig geringen Druck von maximal 33 bar, sogar maximal 28 bar, sogar maximal 24 bar, sogar maximal 19 bar, sogar maximal 18 bar, sogar maximal 17 bar, sogar maximal 16 bar, sogar maximal 15 bar, sogar maximal 14 bar, sogar maximal 13 bar, mit ausreichender Ausbeute durchgeführt werden. Das Verfahren ermöglicht es sogar, die Oxidation des Katalysators mit Luft beim oben genannten Druck ausreichend effizient durchzuführen. Da die Vorrichtung zur Durchführung des Verfahrens dadurch weniger Druck standhalten muss, kann die Vorrichtung deutlich kostengünstiger bereitgestellt werden als eine Vorrichtung zur Durchführung des aus der EP 2 473 467 B1 bekannten Verfahrens zur katalytischen Erzeugung von Ameisensäure.

Unter einem Gas wird hier ein Gas oder ein Gasgemisch verstanden. Unter dem "Volumenanteil von CO und $CO_2$ zusammen" wird die Summe der Volumenanteile von CO und $CO_2$ verstanden. Der bei der Reaktion zur Bildung der Ameisensäure reduzierte Katalysator wird durch Oxidation wieder in seinen Ausgangszustand versetzt. Im Sinne der Erfindung wird unter einem Katalysator also auch ein Stoff verstanden, der während des Verfahrens durch Reduktion verändert und durch Oxidation wieder in seinen Ausgangszustand versetzt wird.

Das bei der Reaktion entstehende und in das Gas übergehende CO und/oder $CO_2$ kann dadurch in einer solchen Menge abgeführt werden, dass der Volumenanteil von CO und $CO_2$ zusammen an dem Gas 80 %, 70 %, 60 %, 55 %, 50 %, 45 %, 40 %, 35 %, 30 %, 25 % oder 20 % nicht übersteigt, dass das die Lösung kontaktierende Gas oder zumindest ein Teil dieses Gases permanent oder stoßweise spätestens bei Erreichen dieses Volumenanteils von 80 %, 70 %, 60 %, 55 %, 50 %, 45 %, 40 %, 35 %, 30 %, 25 % oder 20 % durch frisches Gas ersetzt wird oder indem das CO und/oder das $CO_2$, spätestens bei Erreichen dieses Volumenanteils von 80 %, 70 %, 60 %, 55 %, 50 %, 45 %, 40 %, 35 %, 30 %, 25 % oder 20 %, von dem Gas abgesondert wird. Frisches Gas ist Gas, welches die Lösung bis dahin nicht kontaktiert hat, einen Sauerstoffgehalt von mindestens 18 % aufweist und bei dem der Volumenanteil von CO und $CO_2$ zusammen geringer ist als bei dem ersetzten Gas.

Nachfolgend wird die Erfindung anhand eines Ausführungsbeispiels näher erläutert. Es zeigen:

Fig. 1    eine schematische Darstellung eines erfindungsgemäßen Verfahrens,

Fig. 2    eine Kalibriergerade zur Bestimmung der Konzentration des Katalysators HPA-5 in einer wässrigen Lösung des Katalysators bei einer Wellenlänge von 435 nm und

Fig. 3    eine Kalibriergerade zur Bestimmung der Konzentration des Katalysators HPA-5 in einer wässrigen Lösung des Katalysators bei einer Wellenlänge von 510 nm.

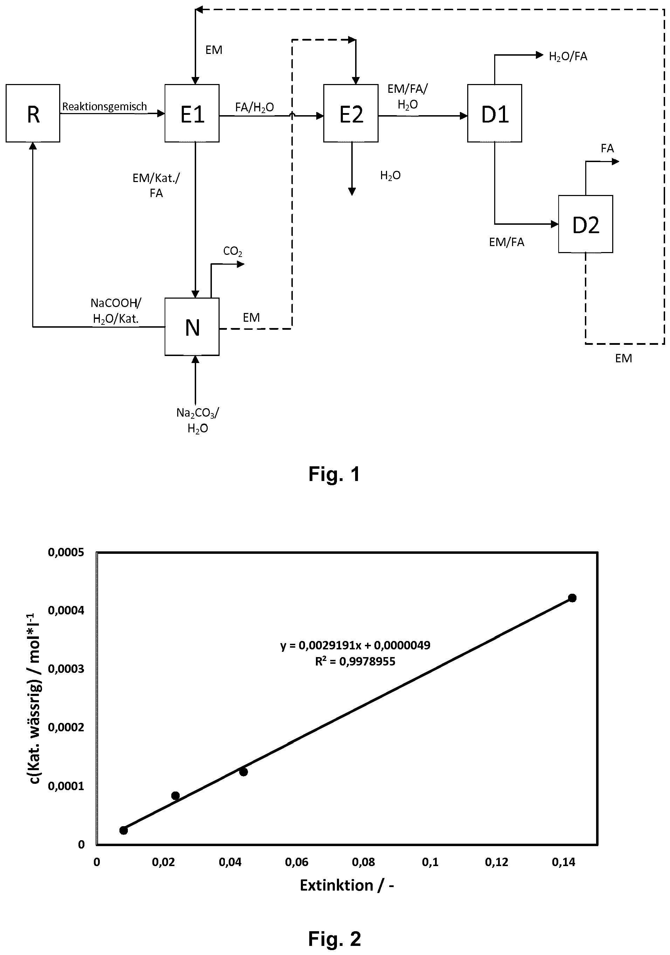

Bei dem Verfahren gemäß Fig. 1 befindet sich in dem Reaktionsgefäß R ein Reaktionsgemisch zur Erzeugung von Ameisensäure FA, welches beispielsweise Glukose und $[PMo_7V_5O_{40}]^{8-}$ enthält. Statt Glukose kann auch ein anderes alpha-Hydroxyaldehyd oder ein anderes der in Patentanspruch 15 genannten Substrate eingesetzt werden. Von dem Reaktionsgefäß R wird eine Teilmenge des Reaktionsgemischs in das erste Extraktionsgefäß E1 geleitet und dort mittels des Extraktionsmittels EM, wie z. B. N,N-Di-n-butylformamid (DBFA), als organische Phase etwa eine Minute lang bei einer Temperatur von etwa 30 °C extrahiert. Dazu genügt eine einstufige Extraktion. Das erste Extraktionsgefäß E1 kann dafür beispielsweise als Mixer-Settler Apparatur aufgebaut sein. Um eine etwa einminütige Extraktion zu erreichen muss dabei die Geschwindigkeit, mit welcher das Extraktionsmittel EM und das Reaktionsgemisch die Mixer-Settler Apparatur durchströmen so gewählt werden, dass die vor allem im Mixer-Bereich der Apparatur erfolgende relevante Kontaktzeit etwa eine Minute beträgt. Aufgrund des deutlich höheren Verteilungskoeffizienten des Extraktionsmittels EM für eine Extraktion des Katalysators als für eine Extraktion der Ameisensäure FA genügt eine solch kurze Extraktionszeit für die Extraktion des Katalysators. Bei der Extraktion geht vor allem der Katalysator, jedoch auch eine geringe Menge der Ameisensäure FA in die organische Phase über.

Nach der Extraktion wird das Extraktionsmittel EM vom ersten Extraktionsgefäß E1 in ein Neutralisationsgefäß N geleitet. Das Extraktionsmittel EM enthält ein unpolares Additiv, wie beispielsweise n-Hexan. In dem Neutralisationsgefäß N wird das Extraktionsmittel bei etwa 90 °C mit einer wässrigen Natriumcarbonatlösung $Na_2CO_3/H_2O$ extrahiert. Dabei bildet sich in der wässrigen Lösung aus der enthaltenen Ameisensäure Natriumformiat $NaCOOH$. Ebenfalls entstehendes $CO_2$ entweicht gasförmig. Gleichzeitig geht der Katalysator durch die Temperaturerhöhung und den Zusatz von Natriumcarbonat in die wässrige Phase über. Die den Katalysator enthaltende wässrige Phase wird wieder in das Reaktionsgefäß R geleitet, so dass der Katalysator für die weitere Umsetzung des Substrats zur Verfügung steht.

Nach Durchführung des ersten Schritts der Extraktion wird die Ameisensäure FA enthaltende wässrige Phase aus dem ersten Extraktionsgefäß E1 in ein zweites Extraktionsgefäß E2 geleitet und dort für 30 Minuten in einer mehrstufigen Extraktion, beispielsweise mittels einer Gegenstromextraktionsapparatur, mit dem Extraktionsmittel EM extrahiert. Bei dem Extraktionsmittel EM kann es sich um das nach der Extraktion mit der wässrigen Natriumcarbonatlösung im Neutralisationsgefäß N verbleibende Extraktionsmittel oder frisches Extraktionsmittel oder eine Mischung daraus handeln. Bei diesem zweiten Schritt der Extraktion geht die Ameisensäure aus der wässrigen Phase in die organische Phase über. Daneben kann sich eine geringe Menge an Wasser $H_2O$ in dem Extraktionsmittel lösen, wenn dieses nicht bereits mit Wasser gesättigt ist. Nach dem zweiten Schritt der Extraktion wird das Extraktionsmittel EM mit darin gelöster Ameisensäure und ggf. gelöstem Wasser $H_2O$ in eine erste Destillationsvorrichtung D1 geleitet, in der Ameisensäure FA und hauptsächlich Wasser $H_2O$ abgesondert werden. Dieses Ameisensäure-Wasser-Gemisch kann beispielsweise zum Ansäuern der aus dem Neutralisationsgefäß hervorgehenden wässrigen Lösung des Katalysators verwendet werden, bevor diese Lösung dem Reaktionsgemisch zugesetzt wird. Ein Ansäuern der Katalysatorlösung ist vorteilhaft, um zu vermeiden, dass der pH-Wert des Reaktionsgemischs in dem Reaktionsgefäß auf über 3, über 2,5 oder über 2 steigt. Ein solcher Anstieg würde zu einer Reduktion der Reaktionsgeschwindigkeit bei der Erzeugung der Ameisensäure führen.

Bei der Destillation kann es sich um eine relativ unkomplizierte Flash-Destillation handeln. Der Rückstand aus der ersten Destillation in der ersten Destillationsvorrichtung D1 kann in eine zweite Destillationsvorrichtung D2 geleitet werden, wo in einer zweiten Destillation, beispielsweise einer zweiten Flash-Destillation, die Ameisensäure vom Extraktionsmittel EM abgesondert wird. Das verbleibende Extraktionsmittel EM kann zur Extraktion in das erste Extraktionsgefäß E1 geleitet werden. Dort kann es ausschließlich oder zusammen mit frischem Extraktionsmittel EM zur Durchführung des ersten Schritts der Extraktion eingesetzt werden.

Der Verteilungskoeffizient kann beispielsweise für den Katalysator HPA-5 gemäß dem folgenden Beispiel ermittelt werden:

In einem Scheidetrichter wurden 40 g N,N-Di-n-butylformamid mit 40 g einer 10 Gew.-% Ameisensäure enthaltenden wässrigen Phase und jeweils verschiedenen Konzentrationen des Katalysators HPA-5 ausgeschüttelt und anschließend eine Woche bei 40 °C stehen gelassen. Dadurch wurde eine vollständige Phasentrennung erreicht. Anschließend wurden die Phasen jeweils getrennt, gewogen und die jeweilige Konzentration an HPA-5 in der wässrigen Phase mittels eines Photometers bei 435 nm gemessen. Der Verteilungskoeffizient K wurde nach der folgenden Formel berechnet:

$$K_{HPA-5} = \frac{Konzentration\ von\ HPA-5\ in\ N,N-di-n-butylformamid}{Konzentration\ von\ HPA-5\ in\ der\ wässrigen\ Phase}$$

Daraus ergaben sich die aus der folgenden Tabelle ersichtlichen Verteilungskoeffizienten bei 40 °C:

| | Extraktionsmittel | c(Kat,Start) [Gew.-%] | c(FA, wässrig) [gew.-%] | c(Kat,w) [mol/m] | c(Kat,org.) [mol/l] | $K_{HPA-5}$ |
|---|---|---|---|---|---|---|
| 1 | DBFA | 1,5 | 0 | $2,25*10^{-6}$ | $7,39*10^{-3}$ | 3284 |

(fortgesetzt)

| | Extraktionsmittel | c(Kat,Start) [Gew.-%] | c(FA, wässrig) [gew.-%] | c(Kat,w) [mol/m] | c(Kat,org.) [mol/l] | $K_{HPA-5}$ |
|---|---|---|---|---|---|---|
| 2 | DBFA | 1,5 | 1 | $2{,}72 * 10^{-6}$ | $7{,}42 * 10^{-3}$ | 2727 |
| 3 | DBFA | 1,5 | 10 | $3{,}18 * 10^{-6}$ | $7{,}05 * 10^{-3}$ | 2216 |

Die Abkürzungen haben dabei die folgenden Bedeutungen:
DBFA: N,N-Di-n-butylformamid; c(Kat,Start): Konzentration des Katalysators HPA-5 am Beginn des Versuchs; c(FA, wässrig): Konzentration der Ameisensäure in der wässrigen Phase; c(Kat,w): Konzentration des Katalysators in der wässrigen Phase; c(Kat,org): Konzentration des Katalysators in der organischen Phase; $K_{HPA-5}$: Verteilungskoeffizient K gemäß obiger Formel.

Die entsprechende Kalibriergerade zur Bestimmung der Konzentration des Katalysators in der wässrigen Phase nach der Extraktion mit DBFA ist in Fig. 2 dargestellt. Dabei ist c(Kat. wässrig) die Konzentration des Katalysators in der wässrigen Phase nach der Phasentrennung.

Weitere Versuche zeigten, dass die Phasentrennung mit einer Ameisensäure neben dem Katalysator enthaltenden wässrigen Phase gegenüber einer Phasentrennung mit einer rein wässrigen Lösung des Katalysators beschleunigt erfolgt.

Bezugszeichenliste

R          Reaktionsgefäß
FA         Ameisensäure
EM         Extraktionsmittel
E1         erstes Extraktionsgefäß
E2         zweites Extraktionsgefäß
D1         erste Destillationsvorrichtung
D2         zweite Destillationsvorrichtung
N          Neutralisationsgefäß
$H_2O$     Wasser
$Na_2CO_3$ Natriumcarbonat
NaCOOH     Natriumformiat

Patentansprüche

1. Verfahren zum Absondern von Ameisensäure aus einem Reaktionsgemisch durch Extraktion, wobei das Reaktionsgemisch neben der Ameisensäure ein Polyoxometallat-Ion der allgemeinen Formel $[PMo_xV_yO_{40}]^{n-}$ als Katalysator und ein den Katalysator lösendes Lösungsmittel umfasst, wobei $6 \leq x \leq 11$, $1 \leq y \leq 6$, $x + y = 12$ und $3 < n < 10$, wobei n, x und y jeweils eine ganze Zahl ist, wobei das Absondern durch eine Extraktion mittels eines die Ameisensäure und den Katalysator extrahierenden polaren organischen Extraktionsmittels erfolgt, welches N-(n-Hexadecyl)formamid, N-Di-n-acetamid oder ein N,N-Dialkylcarbonsäureamid ist, wobei das N,N-Dialkylcarbonsäureamid beim Mischen mit dem Lösungsmittel eine Phasengrenze zwischen dem Lösungsmittel und dem Extraktionsmittel bildet, wobei das Extraktionsmittel ein solches ist, welches für eine Extraktion des in einer Konzentration von 1,5 Gew.-% in Wasser enthaltenen Katalysators bei 40 °C einen Verteilungskoeffizienten für den Katalysator aufweist, welcher mindestens um den Faktor 7 größer ist als ein Verteilungskoeffizient für eine Extraktion der in einer Konzentration von 5 Gew.-% in Wasser enthaltenen Ameisensäure bei 40 °C, wobei das Extraktionsmittel vor der Extraktion mit dem Katalysator gesättigt wird oder wobei der zusammen mit der Ameisensäure extrahierte Katalysator aus dem Extraktionsmittel nach der Extraktion mittels Fällung als Salz oder mittels einer weiteren Extraktion mit einem polaren weiteren Extraktionsmittel und einem Temperaturwechsel des Extraktionsmittels und/oder einer Erhöhung des pH-Werts des Extraktionsmittels abgesondert und wieder dem Reaktionsgemisch zugeführt wird.

2. Verfahren nach Anspruch 1, wobei die Extraktion zweistufig durchgeführt wird, indem das Reaktionsgemisch in einem ersten Schritt der Extraktion mit einer ersten Menge des Extraktionsmittels zur Extraktion des Katalysators und in einem zweiten Schritt der Extraktion mit einer zweiten Menge des Extraktionsmittels zur Extraktion der Ameisensäure extrahiert wird, wobei der in dem ersten Schritt der Extraktion extrahierte Katalysator wieder dem Reaktionsgemisch zugeführt wird, wobei der Katalysator aus dem in dem ersten Schritt der Extraktion eingesetzten Extraktionsmittel nach dem ersten Schritt der Extraktion mittels Fällung als Salz oder mittels einer weiteren Extraktion mit einem polaren weiteren Extraktionsmittel und einem Temperaturwechsel des in dem ersten Schritt der Extraktion eingesetzten Extraktionsmittels und/oder einer Erhöhung des pH-Werts des in dem ersten Schritt der Extraktion eingesetzten Extraktionsmittels abgesondert wird.

3. Verfahren nach Anspruch 2, wobei die erste Menge kleiner ist als die zweite Menge und/oder die Extraktion mit der ersten Menge des Extraktionsmittels für eine erste Zeit und die Extraktion mit der zweiten Menge des Extraktionsmittels für eine zweite Zeit erfolgt und die zweite Zeit länger ist als die erste Zeit.

4. Verfahren nach einem der vorhergehenden Ansprüche, wobei der Katalysator mittels der Fällung als Salz gleichzeitig mit einer Fällung der extrahierten Ameisensäure als Formiat abgesondert wird oder wobei das polare weiter Extraktionsmittel das Lösungsmittel ist und/oder die Erhöhung des pH-Werts durch Zusatz eines Carbonats und/oder eines Hydroxids erfolgt.

5. Verfahren nach einem der vorherigen Ansprüche, wobei die Fällung als Salz mittels eines Hydroxids, insbesondere KOH oder NaOH, oder einer sonstigen Base erfolgt.

6. Verfahren nach einem der vorherigen Ansprüche, wobei das gefällte Salz dem Reaktionsgemisch zugeführt wird und davor in dem Lösungsmittel gelöst wird und, insbesondere mittels Ameisensäure, auf einen pH-Wert des Reaktionsgemischs eingestellt wird und/oder das weitere Extraktionsmittel nach der weiteren Extraktion dem Reaktionsgemisch zugeführt wird und davor, insbesondere mittels Ameisensäure, auf einen pH-Wert des Reaktionsgemischs eingestellt wird.

7. Verfahren nach einem der vorherigen Ansprüche, wobei dem Extraktionsmittel vor der Extraktion oder vor der weiteren Extraktion oder zumindest dem in dem ersten Schritt der Extraktion eingesetzten Extraktionsmittel vor dem ersten Schritt der Extraktion oder vor der weiteren Extraktion ein, insbesondere unpolares, Additiv zugesetzt wird und der Katalysator aus dem Extraktionsmittel nach der Extraktion oder aus dem in dem ersten Schritt der Extraktion eingesetzten Extraktionsmittel nach dem ersten Schritt der Extraktion mittels der weiteren Extraktion mit dem polaren weiteren Extraktionsmittel, insbesondere dem Lösungsmittel, und dem Temperaturwechsel und/oder der Erhöhung des pH-Werts, insbesondere durch Zusatz eines Carbonats und/oder eines Hydroxids, abgesondert wird.

8. Verfahren nach Anspruch 7, wobei das Additiv Petroleum, eine Fraktion von Petroleum, n-Hexan, n-Oktan, n-Decan, Oleylalkohol, Toluol, Dibutylether oder Tri-n-butylphosphat ist.

9. Verfahren nach einem der vorhergehenden Ansprüche, wobei das Lösungsmittel ein protisches und/oder polares Lösungsmittel, insbesondere Wasser oder ein mittels des Katalysators umsetzbares Substrat, ist.

10. Verfahren nach einem der vorhergehenden Ansprüche, wobei das N,N-Dial-kylcarbonsäureamid Dipentylformamid, N,N-Di-n-butylformamid, N-methyl-N-heptylformamid, N-n-butyl-N-2-ethylhexylformamid oder N-n-butyl-N-cyclohexylformamid ist.

11. Verfahren nach einem der vorhergehenden Ansprüche, wobei die Extraktion jeweils nur mit einer Teilmenge des Reaktionsgemischs durchgeführt wird, die nach der Extraktion wieder dem restlichen Reaktionsgemisch zugeführt wird.

12. Verfahren nach einem der vorhergehenden Ansprüche, wobei bei dem zweiten Schritt der Extraktion nur so viel Ameisensäure aus dem Reaktionsgemisch extrahiert wird, dass der pH-Wert des Reaktionsgemischs nicht über 3, insbesondere nicht über 2,5, steigt.

13. Verfahren nach einem der vorhergehenden Ansprüche, wobei das Verfahren eine katalytischen Erzeugung von Ameisensäure mittels des Katalysators und eine Regeneration des dabei reduzierten Katalysators umfasst, wobei der Katalysator bei einer Temperatur oberhalb von 70 °C, 80 °C oder 90 °C und unterhalb von 160 °C, 150 °C oder 140 °C mit einem alpha-Hydroxyaldehyd, einer alpha-Hydroxycarbonsäure, einem Kohlenhydrat, einem Glycosid oder einem eine Kohlenstoffkette enthaltenden Polymer mit mindestens einer wiederholt als Substituent an die Kohlenstoffkette gebundenen OH-Gruppe und/oder mit einem wiederholt in der Kohlenstoffkette enthaltenen O-, N- oder S-Atom als Substrat in dem Reaktionsgemisch in Kontakt gebracht wird, wobei der dabei reduzierte Katalysator durch Oxidation wieder in seinen Ausgangszustand versetzt wird, wobei das Reaktionsgemisch dazu mit einem einen Volumenanteil von mindestens 18 % Sauerstoff enthaltenden Gas bei einem Gasdruck von mindestens 2 bar, mindestens 3 bar, mindestens 4 bar oder mindestens 5 bar und höchstens 33 bar, höchstens 16 bar oder höchstens 15 bar mittels einer Mischvorrichtung oder über eine flüssigkeitsundurchlässige gasdurchlässige Membran in Kontakt gebracht wird, wobei bei der Reaktion entstehendes und in das Gas übergehendes CO und/oder $CO_2$ in einer solchen Menge abgeführt wird, dass ein Volumenanteil von CO und $CO_2$ zusammen an dem Gas 80 %, 70 %, 60 %, 55 %, 50 %, 45 %, 40 %, 35 %, 30 %, 25 % oder 20 % nicht übersteigt.

**Claims**

1. A method for isolating formic acid from a reaction mixture by extraction, wherein the reaction mixture besides the formic acid comprises a polyoxometalate ion of the general formula $[PMo_xV_yO_{40}]^{n-}$ as catalyst and a solvent which dissolves the catalyst, where $6 \leq x \leq 11$, $1 \leq y \leq 6$, $x + y = 12$, and $3 < n < 10$, where n, x and y are each an integer, where the isolating is accomplished by extraction by means of a polar organic extractant which extracts the formic acid and the catalyst and which is N-(n-hexadecyl)formamide, N-di-n-acetamide or an N,N-dialkylcarboxamide, where the N,N-dialkylcarboxamide on mixing with the solvent forms a phase boundary between the solvent and the extractant, where the extractant is one which, for extraction of the catalyst present at a concentration of 1.5 wt% in water, has a partition coefficient for the catalyst at 40°C that is greater by a factor of at least 7 than a partition coefficient for extraction at 40°C of the formic acid present at a concentration of 5 wt% in water, where the extractant before the extraction is saturated with the catalyst or where the catalyst extracted together with the formic acid is isolated from the extractant after the extraction by means of precipitation as a salt or by means of a further extraction with a polar further extractant and with a temperature change of the extractant and/or with an increase in the pH of the extractant, and is returned to the reaction mixture.

2. The method as claimed in claim 1, wherein the extraction is carried out in two stages, by extracting the reaction mixture in a first extraction step with a first quantity of the extractant in order to extract the catalyst and extracting the reaction mixture in a second extraction step with a second quantity of the extractant in order to extract the formic acid, where the catalyst extracted in the first extraction step is returned to the reaction mixture, where the catalyst is isolated from the extractant used in the first extraction step, after the first extraction step, by means of precipitation as a salt or by means of a further extraction with a polar further extractant and with a temperature change of the extractant used in the first extraction step and/or with an increase in the pH of the extractant used in the first extraction step.

3. The method as claimed in claim 2, wherein the first quantity is smaller than the second quantity and/or the extraction with the first quantity of the extractant takes place for a first time and the extraction with the second quantity of the extractant takes place for a second time and the second time is longer than the first time.

4. The method as claimed in any of the preceding claims, wherein the catalyst by means of the precipitation as a salt is isolated simultaneously with precipitation of the extracted formic acid as a formate, or wherein the polar further extractant is the solvent and/or the increase in the pH takes place by addition of a carbonate and/or a hydroxide.

5. The method as claimed in any of the preceding claims, wherein the precipitation as a salt takes place by means of a hydroxide, more particularly KOH or NaOH, or of another base.

6. The method as claimed in any of the preceding claims, wherein the precipitated salt is supplied to the reaction mixture and dissolved beforehand in the solvent and adjusted, in particular by means of formic acid, to a pH of the reaction mixture, and/or the further extractant after the further extraction is supplied to the reaction mixture and adjusted beforehand, in particular by means of formic acid, to a pH of the reaction mixture.

7. The method as claimed in any of the preceding claims, wherein the extractant before the extraction or before the further extraction, or at least the extractant used in the first extraction step, before the first extraction step or before the further extraction, is admixed with an additive, more particularly an apolar additive, and the catalyst is isolated from the extractant after the extraction, or from the extractant used in the first extraction step after the first extraction step, by means of the further extraction with the polar further extractant, more particularly the solvent, and with the temperature change and/or the increase in the pH, more particularly by addition of a carbonate and/or a hydroxide.

8. The method as claimed in claim 7, wherein the additive is petroleum, a fraction of petroleum, n-hexane, n-octane, n-decane, oleyl alcohol, toluene, dibutyl ether or tri-n-butyl phosphate.

9. The method as claimed in any of the preceding claims, wherein the solvent is a protic and/or polar solvent, more particularly water or a substrate which can be reacted by means of the catalyst.

10. The method as claimed in any of the preceding claims, wherein the N,N-dialkylcarboxamide is dipentylformamide, N,N-di-n-butylformamide, N-methyl-N-heptylformamide, N-n-butyl-N-2-ethylhexylformamide or N-n-butyl-N-cyclohexylformamide.

11. The method as claimed in any of the preceding claims, wherein the extraction is carried out in each case only with a portion of the reaction mixture, which after the extraction is returned to the rest of the reaction mixture.

12. The method as claimed in any of the preceding claims, wherein the amount of formic acid extracted from the reaction mixture in the second extraction step is only such that the pH of the reaction mixture does not rise above 3, more particularly not above 2.5.

13. The method as claimed in any of the preceding claims, wherein the method comprises catalytic generation of formic acid by means of the catalyst and regeneration of the catalyst reduced in the process, where the catalyst is contacted at a temperature above 70°C, 80°C or 90°C and below 160°C, 150°C or 140°C with an alpha-hydroxyaldehyde, an alpha-hydroxycarboxylic acid, a carbohydrate, a glycoside or a polymer containing a carbon chain and having at least one OH group bonded repeatedly as substituent to the carbon chain and/or having an O, N or S atom present repeatedly in the carbon chain, as substrate in the reaction mixture, where the catalyst reduced in the process is returned to its original state by oxidation, where the reaction mixture for this purpose is contacted with a gas comprising a volume fraction of at least 18% of oxygen at a gas pressure of at least 2 bar, at least 3 bar, at least 4 bar or at least 5 bar and at most 33 bar, at most 16 bar or at most 15 bar, by means of a mixing apparatus or via a liquid-impermeable, gas-permeable membrane, where CO and/or $CO_2$ formed in the reaction and entering the gas are taken off in a quantity such that the volume fraction of CO and $CO_2$ together in the gas does not exceed 80%, 70%, 60%, 55%, 50%, 45%, 40%, 35%, 30%, 25% or 20%.

## Revendications

1. Procédé de séparation d'acide formique d'un mélange réactionnel par extraction, dans lequel le mélange réactionnel comprend outre l'acide formique un ion polyoxométallate de la formule générale $[PMo_xV_yO_{40}]^{n-}$ en tant que catalyseur et un solvant dissolvant le catalyseur, dans lequel $6 \leq x \leq 11$, $1 \leq y \leq 6$, $x + y = 12$ et $3 < n < 10$, dans lequel n, x et y sont chacun un nombre entier, dans lequel la séparation s'effectue par une extraction au moyen d'un agent d'extraction organique polaire extrayant l'acide formique et le catalyseur qui est le N-(n-hexadécyl)formamide, N-di-n-acétamide ou un amide d'acide N,N-dialkylcarboxylique, dans lequel l'amide d'acide N,N-dialkylcarboxylique forme lors du mélange avec le solvant une limite de phase entre le solvant et l'agent d'extraction, dans lequel l'agent d'extraction est un agent qui présente pour une extraction du catalyseur contenu à une concentration de 1,5 % en poids dans de l'eau à 40°C un coefficient de distribution pour le catalyseur qui est supérieur au moins du facteur 7 à un coefficient de distribution pour une extraction de l'acide formique contenu à une concentration de 5 % en poids dans de l'eau à 40°C, dans lequel l'agent d'extraction est saturé avec le catalyseur avant l'extraction ou dans lequel le catalyseur extrait avec l'acide formique est séparé de l'agent d'extraction après l'extraction au moyen d'une précipitation en tant que sel ou au moyen d'une autre extraction avec un autre agent d'extraction polaire et un changement de température de l'agent d'extraction et/ou une augmentation de la valeur de pH de l'agent d'extraction et ramené au mélange réactionnel.

2. Procédé selon la revendication 1, dans lequel l'extraction est réalisée en deux étapes en ce que le mélange réactionnel est extrait dans une première étape de l'extraction avec une première quantité de l'agent d'extraction en vue de l'extraction du catalyseur et dans une seconde étape de l'extraction avec une seconde quantité de l'agent d'extraction en vue de l'extraction de l'acide formique, dans lequel le catalyseur extrait dans la première étape de l'extraction est ramené au mélange réactionnel, dans lequel le catalyseur est séparé de l'agent d'extraction utilisé dans la première étape de l'extraction après la première étape de l'extraction au moyen d'une précipitation en tant que sel ou au moyen d'une autre extraction avec un autre agent d'extraction polaire et un changement de température de l'agent d'extraction utilisé dans la première étape de l'extraction et/ou une augmentation de la valeur de pH de l'agent d'extraction utilisé dans la première étape de l'extraction.

**3.** Procédé selon la revendication 2, dans lequel la première quantité est inférieure à la seconde quantité et/ou l'extraction avec la première quantité de l'agent d'extraction s'effectue pendant une première durée et l'extraction avec la seconde quantité de l'agent d'extraction s'effectue pendant une seconde durée, et la seconde durée est plus longue que la première durée.

**4.** Procédé selon une des revendications précédentes, dans lequel le catalyseur est séparé au moyen de la précipitation en tant que sel simultanément avec une précipitation de l'acide formique extrait en tant que formiate ou dans lequel l'autre agent d'extraction polaire est le solvant et/ou l'augmentation de la valeur de pH s'effectue par ajout d'un carbonate et/ou d'un hydroxyde.

**5.** Procédé selon une des revendications précédentes, dans lequel la précipitation en tant que sel s'effectue au moyen d'un hydroxyde, notamment KOH ou NaOH, ou d'une autre base.

**6.** Procédé selon une des revendications précédentes, dans lequel le sel précipité est amené au mélange réactionnel et est dissous auparavant dans le solvant et est réglé, notamment au moyen d'acide formique, sur une valeur de pH du mélange réactionnel et/ou l'autre agent d'extraction est amené au mélange réactionnel après l'autre extraction et est réglé auparavant, notamment au moyen d'acide formique, sur une valeur de pH du mélange réactionnel.

**7.** Procédé selon une des revendications précédentes, dans lequel un additif, notamment non polaire, est ajouté à l'agent d'extraction avant l'extraction ou avant l'autre extraction ou au moins à l'agent d'extraction utilisé dans la première étape de l'extraction avant la première étape de l'extraction ou avant l'autre extraction, et le catalyseur est séparé de l'agent d'extraction après l'extraction ou de l'agent d'extraction utilisé dans la première étape de l'extraction après la première étape de l'extraction au moyen de l'autre extraction avec l'autre agent d'extraction polaire, notamment le solvant, et le changement de température et/ou l'augmentation de la valeur de pH, notamment par ajout d'un carbonate et/ou d'un hydroxyde.

**8.** Procédé selon la revendication 7, dans lequel l'additif est le pétrole, une fraction de pétrole, le n-hexane, n-octane, n-décane, l'alcool oléylique, le toluène, l'éther dibutylique ou le tri-n-butylphosphate.

**9.** Procédé selon une des revendications précédentes, dans lequel le solvant est un solvant protique et/ou polaire, notamment de l'eau ou un substrat pouvant être converti au moyen du catalyseur.

**10.** Procédé selon une des revendications précédentes, dans lequel l'amide d'acide N,N-dialkylcarboxylique est le dipentylformamide, N,N-di-n-butylformamide, N-méthyl-N-heptylformamide, N-n-butyl-N-2-éthylhexylformamide ou N-n-butyl-N-cyclohexylformamide.

**11.** Procédé selon une des revendications précédentes, dans lequel l'extraction est réalisée à chaque fois uniquement avec une quantité partielle du mélange réactionnel qui est ramenée après l'extraction au mélange réactionnel restant.

**12.** Procédé selon une des revendications précédentes, dans lequel seule une quantité suffisante d'acide formique n'est extraite du mélange réactionnel lors de la seconde étape de l'extraction pour que la valeur de pH du mélange réactionnel n'augmente pas au-dessus de 3, notamment pas au-dessus de 2,5.

**13.** Procédé selon une des revendications précédentes, dans lequel le procédé comprend une génération catalytique d'acide formique au moyen du catalyseur et une régénération du catalyseur réduit ce faisant, dans lequel le catalyseur est amené en contact dans le mélange réactionnel à une température au-dessus de 70°C, 80°C ou 90°C et en dessous de 160°C, 150°C ou 140°C avec un alpha-hydroxyaldéhyde, un acide alpha-hydroxycarboxylique, un hydrate de carbone, un glycoside ou un polymère contenant une chaîne de carbones avec au moins un groupe OH fixé de manière répétée en tant que substituant à la chaîne de carbones et/ou avec un atome de O, N ou S contenu de manière répétée dans la chaîne de carbones en tant que substrat, dans lequel le catalyseur réduit ce faisant est remis dans son état de départ par oxydation, dans lequel le mélange réactionnel est amené en contact à cet effet avec un gaz contenant une proportion volumique d'au moins 18 % d'oxygène à une pression gazeuse d'au moins 2 bars, d'au moins 3 bars, d'au moins 4 bars ou d'au moins 5 bars et d'au plus 33 bars, d'au plus 16 bars ou d'au plus 15 bars au moyen d'un dispositif de mélange ou par le biais d'une membrane imperméable au liquide, perméable au gaz, dans lequel du CO et/ou $CO_2$ apparaissant lors de la réaction et passant dans le gaz est évacué en une quantité telle qu'une proportion volumique de CO et $CO_2$ ensemble dans le gaz ne dépasse pas 80 %, 70 %, 60 %, 55 %, 50 %, 45 %, 40 %, 35 %, 30 %, 25 % ou 20 %.

**Fig. 1**

**Fig. 2**

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102011077232 A1

- EP 2473467 B1

**In der Beschreibung aufgeführte Nicht-Patentliteratur**

- **GEMÄß SCHIERBAUM B. et al.** Integriertes Verfahren zur Reinigung von carbonsäurehaltigen Prozeßabwässern. *Chemie Ingenieur Technik,* 1997, vol. 69, 519-523